# EUROPEAN PATENT SPECIFICATION

(11) **EP 3 372 214 B1**
(45) Date of publication and mention of the grant of the patent: **30.10.2019**
(21) Application number: 16881535.5
(22) Date of filing: 02.11.2016
(51) Int. Cl.: A61F 13/56, A61F 13/496

(54) **ABSORBENT ARTICLE**
SAUGFÄHIGER ARTIKEL
ARTICLE ABSORBANT

(30) Priority: 28.12.2015 JP 2015256833
(43) Date of publication of application: 12.09.2018
(73) Proprietor: Unicharm Corporation, Ehime 799-0111 (JP)
(72) Inventor: SAITO, Kyota, Kanonji-shi Kagawa 769-1602 (JP); CHANGCHAROEN, Jirapa, Chachoengsao (TH); PICHADKITJAWAT, Sarinee, Chachoengsao (TH)
(74) Representative: Dolleymores
(86) International application number: PCT/JP2016/082569
(87) International publication number: WO 2017/115555

(56) References cited:
- WO-A1-2007/073247
- WO-A1-2016/189904
- JP-A- 2008 104 874
- JP-A- 2008 104 874
- JP-U- H 045 826
- JP-U- 3 082 938
- US-A- 5 368 585

## Description

### [Technical Field]

The present invention relates to an absorbent article.

### [Background Art]

A disposable diaper such as a so-called tape-type diaper or an underpants-shaped disposable diaper is conventionally known as an absorbent article for absorbing excrement. These tape-type diapers and underpants-shaped disposable diapers have a problem of difficulty in being put on a wriggling infant and a problem that the diapers force a wearer to take an unnatural posture when being put on. In order to solve these problems, PTL 1 discloses a half-open underpants-shaped diaper in which portions on either one side of the left or right sides of the waist part are joined together, and portions on the other side are not joined together.

### [Citation List]

### [Patent Literature]

[PTL 1] Japanese Unexamined Utility Model Application Publication No. H 4-5826

JP 2008 104874 A discloses a diaper with a leg opening formed on one side by heat sealing of the front part and the back part of the diaper. On the other side, a fastening tape is provided on the back part to be removably connected to a target tape on the front part.

### [Summary of Invention]

### [Technical Problem]

When the half-open underpants-shaped diaper is to be put on, one leg of the wearer is first inserted into the leg hole of either one side of the left or right sides of the waist part. Thereafter, the front and back waist parts on the other side are pulled to the other side together so as to overlap, and a fastening portion (tape fastener; provided on the back waist part side) is joined to a target region (adhesion region; provided on the front waist part side).

In such an operation for putting on a diaper, when pulling the front and back waist parts to the other side, the position of a diaper is adjusted relative to the wearer's body. But, in case of pulling the waist part to only a one side (the other side), it is difficult to adjust the position of the absorbent body to be aligned with the center of the wearer's body. In a half-open, underpants-shaped diaper of PTL 1, though the shapes of components such as the waist portion is asymmetric on the left and right sides, the diaper is designed based on a symmetric configuration similar to a conventional diaper. Consequently, when a diaper is put on, the position of its absorbent body is not aligned with the center, causing a problem of leakage of excrement or a problem that the fit of the diaper deteriorates due to the diaper being unbalanced on the left and right.

The present invention was achieved in light of the problems described above, and an aspect of the present invention is to provide a half-open diaper which is easily balanced on the left and right sides and has a good fit when being put on. This makes it easier to adjust the position of the absorbent body to be aligned with the center of the wearer's body and makes it easier to balance the diaper on the left and right. Accordingly, it is possible to give the good fit to the wearer.

### [Solution to Problem]

The present invention provides the absorbent article of independent claim 1. The dependent claims specify preferred but optional features.

An aspect of the invention to achieve the above advantage is an absorbent article having a longitudinal direction, a lateral direction intersecting the longitudinal direction, and a front-back direction intersecting the longitudinal direction and the lateral direction, the absorbent article including: a front waist portion extending along the lateral direction; a back waist portion extending along the lateral direction; and an absorbent main body including an absorbent body that absorbs liquid, and being provided between the front waist portion and the back waist portion, a one-side end portion of the back waist portion on a one side in the lateral direction being joined by a first joining portion to a one-side end portion of the front waist portion on the one side in the lateral direction, the back waist portion including a fastening portion on another side in the lateral direction, the fastening portion being fastened to the front waist portion when putting on the absorbent article, the front waist portion having a first region located on the one side relative to a lateral center of the absorbent main body, the front waist portion having a second region located on the other side relative to the lateral center of the absorbent main body, an elastic region stretching/contracting in the lateral direction, the elastic region being provided in at least a part of the first region, the elastic region being not provided in a region of the second region on the other side relative to another-side end of the absorbent main body on the other side.

Other features of the present invention will become apparent from the description of the present specification and the accompanying drawings.

### [Advantageous Effects of Invention]

According to the invention, it is possible to provide a half-open diaper which is easily balanced on the left and right sides and has a good fit when being put on.

### [Brief Description of Drawings]

FIG. 1 is a schematic perspective view of a diaper 1 according to an embodiment.
FIG. 2 is a plan view of the diaper 1 in an unfolded state.
FIG. 3 is a diagram illustrating elastic regions X and Y.
FIG. 4 is a diagram illustrating a method of putting on the diaper 1.
FIG. 5 is a diagram illustrating a force exerted on the waist portion 30 when the diaper 1 is put on.
FIG. 6 is a diagram illustrating a force exerted on the waist portion 30 when the diaper according to a modified example is put on.
FIG. 7 is a diagram illustrating a force exerted on the back waist portion 20 when the diaper 1 is put on.
FIG. 8 is a schematic sectional view illustrating continuity between an elastic region X3 and an elastic region Y.

### [Description of Embodiments]

At least the following matters will be made clear by the description in the present specification and the accompanying drawings.

An absorbent article having a longitudinal direction, a lateral direction intersecting the longitudinal direction, and a front-back direction intersecting the longitudinal direction and the lateral direction, the absorbent article including: a front waist portion extending along the lateral direction; a back waist portion extending along the lateral direction; and an absorbent main body including an absorbent body that absorbs liquid, and being provided between the front waist portion and the back waist portion, a one-side end portion of the back waist portion on a one side in the lateral direction being joined by a first joining portion to a one-side end portion of the front waist portion on the one side in the lateral direction, the back waist portion including a fastening portion on another side in the lateral direction, the fastening portion being fastened to the front waist portion when putting on the absorbent article, the front waist portion having a first region located on the one side relative to a lateral center of the absorbent main body, the front waist portion having a second region located on the other side relative to the lateral center of the absorbent main body, an elastic region stretching/contracting in the lateral direction, the elastic region being provided in at least a part of the first region, the elastic region being not provided in a region of the second region on the other side relative to another-side end of the absorbent main body on the other side.

With such an absorbent article, in the operation for putting on the absorbent article, when the front waist portion is pulled in the lateral direction, a region of the second region located laterally on the other side relative to the absorbent main body does not stretch/contract. This makes it easier to move the absorbent main body in the lateral direction with a force for pulling the front waist portion. Since the first region does stretch/contract, a force for pulling the absorbent main body is exerted in a direction opposite thereto, the absorbent main body enlarges in the lateral direction.

In such an absorbent article, it is desirable
that the back waist portion has a third region located on the one side relative to the lateral center of the absorbent main body, and
that the elastic region is provided in at least a part of the third region.

With such an absorbent article, in the operation for putting on the absorbent article, when the back waist portion is pulled in the lateral direction, a lateral stretching force is produced by the elastic region of the third region, pulling the absorbent main body in a direction opposite thereto. Accordingly, the absorbent main body enlarges in the lateral direction, making it difficult to produce creases or the like on the absorbent body. This makes it possible to improve the fit of the absorbent article when being put on.

In such an absorbent article, it is desirable
that the back waist portion has a fourth region located on the other side relative to the lateral center of the absorbent main body, and
that the elastic region is provided in at least a part of the fourth region.

With such an absorbent article, a lateral stretching force is exerted on a wide range of the back waist portion 20. This makes it easier to fit the entirety of the back waist portion 20 to the wearer's body with the absorbent main body enlarging in the lateral direction. In particular, even in the wearer's buttocks having a large area, it is difficult for the absorbent article to shift.

In such an absorbent article, it is desirable
that the elastic region is not provided in a region located on the other side relative to the elastic region that is provided in the fourth region.

With such an absorbent article, a part to be held for pulling the back waist portion in the lateral direction when putting on the absorbent article (the fastening member) does not stretch/contract, making it easy to handle the part. In addition, since the fastening portion is provided in such a non-stretching/contracting region, this suppress the fastening portion from creasing, making it easier to maintain its planer shape. Accordingly, it is possible to securely fasten the fastening portion to the front waist portion (the target region).

In such an absorbent article, it is desirable
that the elastic region provided in the third region and the elastic region provided in the fourth region are continuous in the lateral direction to each other.

With such an absorbent article, the stretching force of the elastic region provided in the third region and the stretching force of the elastic region provided in the fourth region work together, ensuring stretchability throughout the entirety of the back waist portion. This makes it easier for the stretching forces to be exerted evenly. This makes it difficult for the back waist portion to cause local variation of the stretching proportion and distortion, improving the fit of the absorbent article when it is put on. Also, the back waist portion becomes likely to fit on wearer's back.

In such an absorbent article, it is desirable
that the elastic region provided in the first region and the elastic region provided in the third region are substantially continuous in the lateral direction to each other.

With such an absorbent article, the stretching force of the elastic region provided in the third region and the stretching force of the elastic region provided in the first region work together, ensuring stretchability throughout the entire circumference of the waist opening. This makes it easier for the stretching forces to be exerted evenly. This makes it difficult for the waist opening to cause local variation of the stretching proportion and distortion, improving the fit of the absorbent article when it is put on. Also, this improves the fit of the absorbent article when it is put on.

In such an absorbent article, it is desirable
that the elastic region is not provided at all in any region of the second region.

With such an absorbent article, in the operation for putting on the absorbent article, when the front waist portion is pulled in the lateral direction, the second region does not stretch/contract as a whole. This makes it easier for a force for pulling the front waist portion to be exerted directly on the absorbent body, making it easier to shift the absorbent body in the lateral direction. This makes it easier for the position of the absorbent body to fit precisely to the center of the wearer's body.

In such an absorbent article, it is desirable
that on a non-skin side of the front waist portion, a target region is provided in a predetermined region of the second region, and
that the target region is a region to which the fastening portion is fastened.

With such an absorbent article, the entirety of the second region is difficult to stretch/contract. Accordingly, the target region is likely to maintain its plain shape, and is less likely to crease. Further, the fastening portion is easy to be fastened to the surface of the target region, and after fastening, these components are difficult to be pulled apart. This makes it difficult for the absorbent article to shift even when the wearer moves his/her body, making it easier to maintain the good fit.

In such an absorbent article, it is desirable
that a leg-gather elastic member is provided on each of lateral end portions of a crotch portion, the leg-gather elastic member being capable of stretching/contracting in the longitudinal direction, and
that a range on which an elastic force of the leg-gather elastic member is exerted overlaps in the longitudinal direction at least a partial region of the back waist portion on the other side relative to the lateral center of the absorbent main body.

With such an absorbent article, in the operation for putting on the absorbent article, when the back waist portion is pulled in the lateral direction, the leg-gather elastic member is pulled together with the back waist portion, pulling obliquely upward the leg gather. Also, stretching the leg gather makes it difficult to crease, making it possible to improve the fit around legs on the wearer's back (buttocks) side.

In such an absorbent article, it is desirable that a longitudinal length of the back waist portion is larger than a longitudinal length of the front waist portion.

With such an absorbent article, it is possible for the back waist portion to reliably cover the wearer's buttocks having a large area when putting on the absorbent article.

### Embodiments

### Basic Configuration of Disposable diaper 1

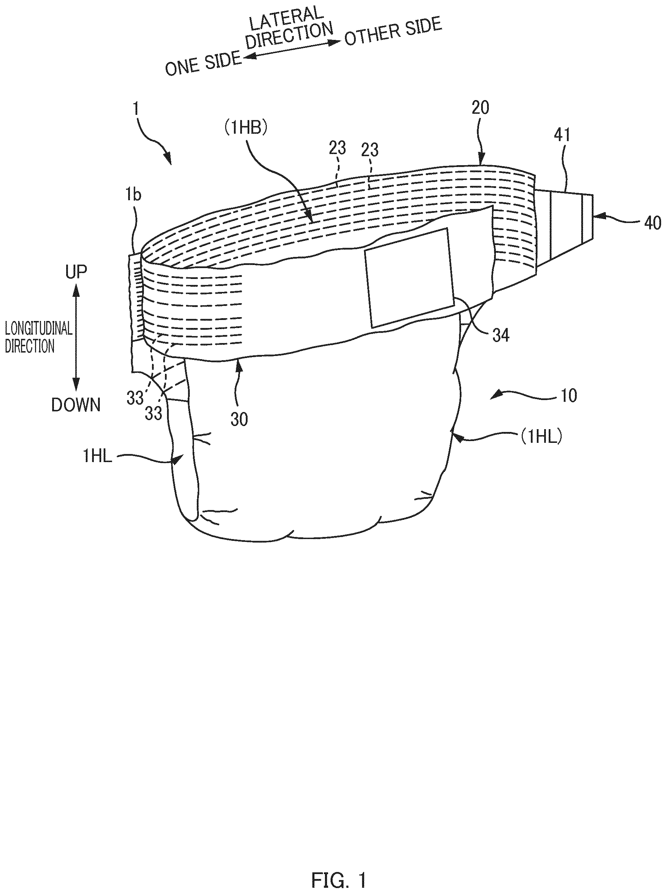

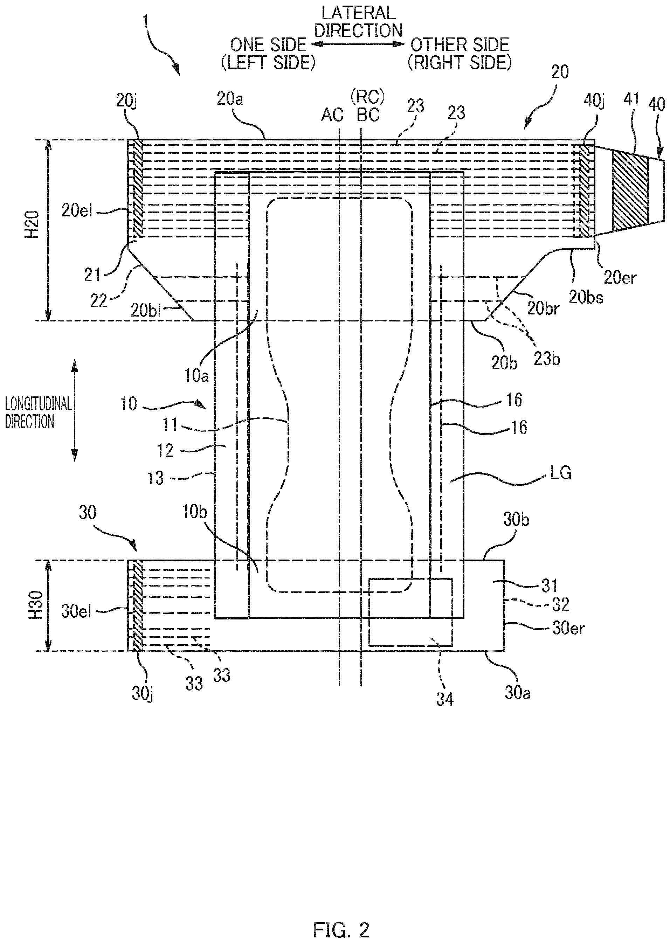

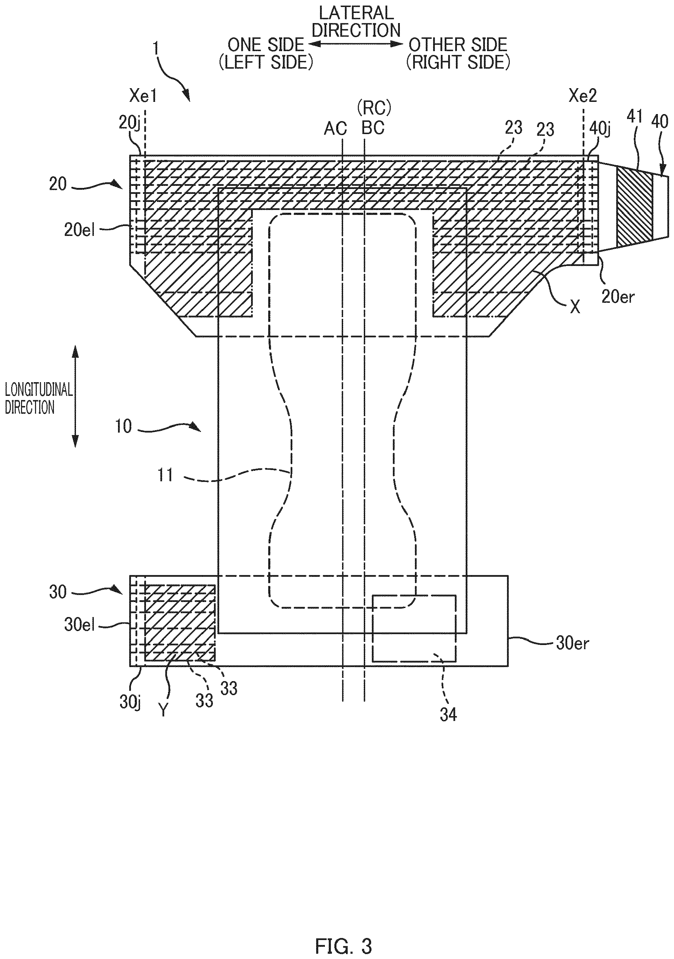

The following describes the configuration of a half-open disposable diaper 1 (hereinafter, also simply called the diaper 1) as an example of an absorbent article according to the present embodiment. FIG. 1 is a schematic perspective view of the diaper 1 according to the present embodiment. FIG. 2 is a plan view of the diaper 1 in an unfolded state. The unfolded state is a state where a fastening portion 41 of the diaper 1 in FIG. 1 is detached from a target region 34, a first joining portion 1b is uncoupled and opened, and the entirety of the diaper 1 is laid flat. FIG. 3 is a diagram illustrating elastic regions X and Y.

The diaper 1 according to the present embodiment is a disposable diaper mainly for use of newborn infants, infants, or the like. As shown in FIG. 1, the diaper 1 has a "longitudinal direction", a "lateral direction" that intersects the longitudinal direction, and a "front-back direction" that intersects the longitudinal direction and the lateral direction. Also, in the following description, the left side in the lateral direction is the "one side", and the right side is the "other side" (see FIGS. 1 and 2).

The diaper 1 includes: an absorbent main body 10 (also called a "crotch portion") that is arranged at the crotch of the wearer and absorbs excrement; a back waist portion 20 that covers the back side of the wearer; and a front waist portion 30 and a fastening member 40 that cover the stomach side of the wearer. The diaper 1 in the unfolded state shown in FIG. 2 is folded in half with an approximately central position in the longitudinal direction serving as the folding position, and end portions of the back waist portion 20 and the front waist portion 30 on one side in the lateral direction are joined together in the first joining portion 1b, thus forming a leg opening 1HL on one side. The end portions of the back waist portion 20 and the front waist portion 30 on the other side in the lateral direction are not joined together, and a leg opening 1HL on the other side and a waist opening 1HB are formed by fastening the fastening member 40 to the front waist portion 30. In other words, the diaper 1 of the present embodiment is a so-called "half-open diaper" in which the back waist portion 20 and the front waist portion 30 are joined and closed on the one side in the lateral direction, and are unjoined and open on the other side.

The back waist portion 20 and the front waist portion 30 have an approximately rectangular planar shape, and in the unfolded state in FIG. 2, the back waist portion 20 and the front waist portion 30 are parallel with a space between each other in the longitudinal direction, and are bridged by the absorbent main body 10. Also, the back waist portion 20 is fixed to one end portion 10a of the absorbent main body 10, and the front waist portion 30 is fixed to an other end portion 10b.

### Absorbent Main Body 10

The absorbent main body 10 (crotch portion) is approximately shaped as an elongated rectangle in a plan view, and is arranged at a central position in the lateral direction, with its lengthwise direction extending along the longitudinal direction of the diaper 1. In FIG. 2, a center line AC is the center of the absorbent main body 10 in the lateral direction. The absorbent main body 10 includes: an absorbent body 11 that absorbs and holds a liquid; a liquid-permeable top face sheet 12 that covers the absorbent body 11 on the wearer's skin side and allows the passage of excrement such as urine; and a liquid-impermeable back-face sheet 13 that covers the absorbent body 11 on the non-skin side and prevents the leakage of a liquid from the non-skin side. The absorbent body 11 is constituted by liquid-absorbent fibers such as pulp fibers and is formed with a predetermined shape such as approximately an hourglass shape in a plan view as shown in FIG. 2, and has a superabsorbent polymer incorporated therein.

Also, leg gathers LG (leg elastic portions) that stretch and contract along the longitudinal direction are provided at locations on respective side portions in the lateral direction of the absorbent main body 10. The leg gathers LG are formed by a nonwoven fabric or the like, and each include a leg-gather elastic member 16 that stretches and contracts along the longitudinal direction. The leg-gather elastic members 16 are formed by an elastic string or the like, and are joined to the leg gathers LG with being stretched in the longitudinal direction, giving the leg gathers LG stretchability in the longitudinal direction. Also, a pair of leg side gathers LSG (barrier cuffs) may be further provided inward of the leg gathers LG in the lateral direction of the absorbent main body 10 (not shown in FIG. 2).

### Back Waist Portion 20

The back waist portion 20 includes: a skin-side member 21 that is located on the wearer's skin side; a non-skin-side member 22 that is located on the non-skin side; and a plurality of elastic strings 23 that are located between the skin-side member 21 and the non-skin-side member 22. The skin-side member 21 and the non-skin-side member 22 are each a flexible sheet member that is constituted by a nonwoven fabric or the like. The elastic strings 23 are elastic members that give the back waist portion 20 lateral stretchability. In the present embodiment, the elastic strings 23 are arranged side-by-side at a predetermined longitudinal interval. And the elastic strings 23 are joined with an adhesive between the skin-side member 21 and the non-skin-side member 22 with being stretched in the lateral direction.

In FIG. 2, a center line RC indicates the lateral center of the back waist portion 20. A joining region 20j is provided in an end portion on the one side of the back waist portion 20. The joining region 20j is joined to a joining region 30j (described later) of the front waist portion 30 by certain joining means (e.g., heat welding), forming a first joining portion 1b of the diaper 1. Also, a fixing region 40j is provided in an end portion on the other side of the back waist portion 20. The fastening member 40 is fixed to the fixing region 40j.

The elastic strings 23 form an elastic region X and improve the fit of the diaper 1. In the upper end portion of the back waist portion 20, the elastic strings 23 are continuous from the one end side to the other end side in the lateral direction, and are arranged side-by-side at a predetermined longitudinal interval. Here, in the longitudinal central portion and the lower longitudinal end portion of the back waist portion 20, the back waist portion 20 is overlapped with the absorbent body 11. In that region, in the widthwise central portion of the back waist portion 20, in which the absorbent body 11 is provided, the elastic strings 23 are not provided. On the other hand, in the respective regions to the left and right of the absorbent body 11, the elastic strings 23 are provided substantially parallel with each other.

As shown in FIG. 3, the elastic region X is the region where the elastic strings 23 are provided, and extends from the laterally inward end (the end on the other side) of the joining region 20j to the laterally inward end (the end on the one side) of the fixing region 40j; as mentioned above, in the lateral direction, the joining region 20j is provided on the one side of the back waist portion 20, and the fixing region 40j is provided on the other side. In FIG. 3, the elastic region X is indicated by the hatched portion. Note that for convenience in FIG. 3, hatching has been omitted for the joining regions 20j and 30j and the fixing region 40j. As shown in FIGS. 2 and 3, the elastic strings 23 extend to lateral ends 20er and 20el of the back waist portion 20. But, providing the joining region 20j and the fixing region 40j respectively in these lateral end portions of the back waist portion 20 makes it substantially impossible to exhibit stretching force of the following regions: the joining region 20j; the portion laterally outward of the joining region 20j; the fixing region 40j; and the portion laterally outward of the fixing region 40j. A one-side end Xe1 of the elastic region X is at the same position as the laterally inward end of the joining region 20j, and an other-side end Xe2 of the elastic region X is at the same position as the laterally inward end of the fixing region 40j. Note that a configuration is possible in which the elastic strings 23 are also provided in a region other than elastic region X indicated by the hatched portion in FIG. 3 (a non-elastic region), and the elastic strings 23 are cut in this non-elastic region to prevent stretchability from being exhibited.

A center line BC indicates the approximate center of the body of a wearer when a wearer's leg has been inserted. In the present embodiment, the center line BC is at the same position as the center line RC that indicates the lateral center of the back waist portion 20. The center line RC and the center line BC are located different from the center line AC that indicates the center of the absorbent main body 10, and are to the right (on the other side) of the center line AC. The reason for this will be described later.

In the back waist portion 20, on the one side of its lower end 20b, provided is an inclined portion 20bl that is inclined toward the one-side end portion. On the other side of the lower end 20b, a straight portion 20bs and an inclined portion 20br are provided; the straight portion 20bs is substantially parallel with the lower end of the front waist portion 30, and the inclined portion 20br is on the laterally inward side of the straight portion 20bs and is symmetrical with the inclined portion 20bl about the center line AC. Thus, providing the right and left inclined portions in a symmetrical manner makes it easier for both right and left inclined portions to fit the roundness of a wearer's buttocks. Due to the straight portion 20bs being substantially parallel with the lower end of the front waist portion 30, when the diaper 1 is put on by fastening the fastening portion 41 to the target region 34, the lower end of the front waist portion 30 can be aligned with the straight portion 20bs of the back waist portion 20. This improves the appearance of the diaper 1 when it is worn. As shown in FIG. 2, the distance in the longitudinal direction (longitudinal length) from the upper end 20a of the back waist portion 20 to the lower end 20b is defined as a length H20.

### Fastening Member 40

The fastening member 40 is a tape substrate having a substantially trapezoidal shape, and is fixed to the back waist portion 20 in the fixing region 40j using a predetermined fixing means such as heat welding. The fastening member 40 has a fastening portion 41, which is a hook-and-loop fastener having a plurality of fastening projections (hooks) on its skin-side surface. The fastening projections of the fastening portion 41 are hooked to the target region 34 provided in the front waist portion 30, fastening the fastening member 40 to the front waist portion 30. The leg opening 1HL on the other side, and the waist opening 1HB are thus formed (see FIG. 1). When the diaper 1 is put on, if the fastening portion 41 is pulled in the lateral direction according to contraction of the elastic region X, the fastening projections (hooks) becomes more likely to be caught by the target region 34. This makes it possible for the fastening portion 41 to be fastened more securely.

Note that in the example shown in FIG. 2, the back waist portion 20 and the fastening member 40 are formed as separate members, and are joined to each other in the fixing region 40j. But, the back waist portion 20 and the fastening member 40 may be formed together as a single unit. Specifically, the front waist portion 30 and the fastening member 40 in FIG. 2 may be combined into the "back waist portion 20". In this case, the fastening portion 41 is directly joined to the laterally, other-side end portion of the back waist portion 20. In this case, the elastic region X is provided such that the other-side end Xe2 of the elastic region X is located inside (laterally closer to the one side than) the laterally, one-side end portion of the fastening portion 41.

### Front Waist Portion 30

The front waist portion 30 includes: a skin-side member 31 that is located on the wearer's skin side; a non-skin-side member 32 that is located on the non-skin side; a plurality of elastic strings 33 (elastic members) that are located between the skin-side member 31 and the non-skin-side member 32; and the target region 34 on the non-skin-side surface of the front waist portion 30. The skin-side member 31 and the non-skin-side member 32 are each a flexible sheet member that is constituted by a nonwoven fabric or the like. The elastic strings 33 are elastic members that give the front waist portion 30 lateral stretchability. In the present embodiment, the elastic strings 33 are arranged side-by-side at a predetermined longitudinal interval. And, the elastic strings 33 are joined with an adhesive between the skin-side member 31 and the non-skin-side member 32 with being stretched in the lateral direction. The target region 34 is a region capable of engaging with the fastening portion 41. For example, the target region 34 is made of a member whose fibers on the upper surface of a nonwoven fabric are formed into loop shapes so as to be readily engaged with the fastening projections (hooks) of the fastening portion 41. Note that the following configuration is also acceptable: instead of making the target region 34 and the front waist portion 30 different, the target region 34 is formed by processing a partial region of the non-skin-side member 32 of the front waist portion 30.

The elastic strings 33 form an elastic region Y, improving the fit of the diaper 1. The elastic strings 33 extend from a laterally, one-side (left) end 30el of the front waist portion 30 to a predetermined lateral position that is located on the one side relative to the center line AC. In the present embodiment, the elastic strings 33 (the elastic region Y) are provided in a range extending from the lateral end 30el to a position laterally on the one side relative to the absorbent main body 10. In contrast, the elastic strings 33 are not provided in the range from a laterally, other-side (right) end 30er to the center line AC. Also, as shown by a hatched portion in FIG. 3, the elastic region Y is a region in which the elastic strings 33 are provided from the laterally inward end of the joining region 30j to the left end 10el of the absorbent main body 10. Note that a configuration is possible in which the elastic strings 33 are also provided in a region other than elastic region Y indicated by the hatched portion in FIG. 3 (a non-elastic region), and the elastic strings 33 are cut in this non-elastic region to prevent stretchability from being exhibited.

As shown in FIG. 2, the joining region 30j is provided in the left end portion of the front waist portion 30. The joining region 30j is joined with the joining region 20j of the back waist portion 20, forming the first joining portion 1b. Also, the lateral length of the front waist portion 30 is smaller than lateral length of the back waist portion 20. Specifically, in the lateral direction, the one-side (left) end 30el of the front waist portion 30 is provided at substantially the same position as the one-side (left) end 20el of the back waist portion 20. In contrast, in the lateral direction, the other-side (right) end 30er of the front waist portion 30 is provided inside the other-side (right) end 20er of the back waist portion 20.

As shown in FIG. 2, the distance in the longitudinal direction (longitudinal length) from the upper end 30a of the front waist portion 30 to the lower end 30b is defined as a length H30.

### Method of Putting on Diaper 1

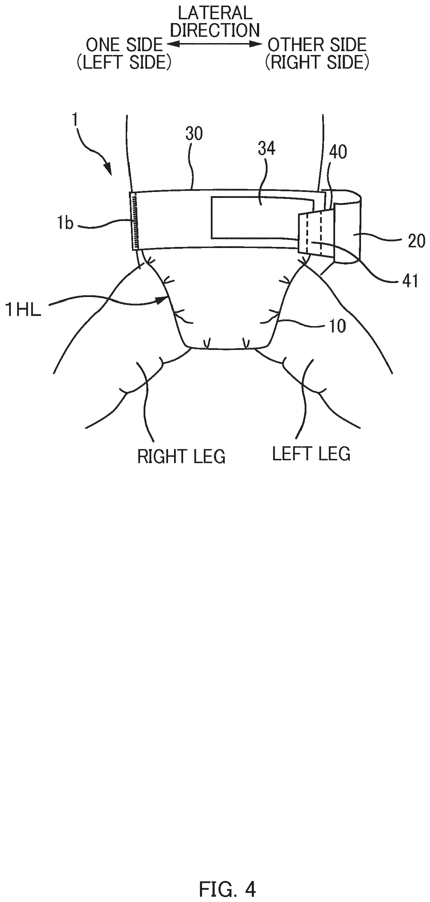

FIG. 4 is a diagram illustrating a method of putting on the diaper 1. As described above, in the laterally one-side end portion of the diaper 1, the front waist portion 30 and the back waist portion 20 are joined together by the first joining portion 1b, thus forming the leg opening 1HL. In contrast, the laterally, other-side end portion is in an open state in which the front waist portion 30 and the back waist portion 20 are not joined together. In other words, the diaper 1 is in a state where its lateral one side is open. When this diaper 1 is to be put on the wearer (an infant or the like), first, one leg (the right leg) of the wearer is inserted into the leg opening 1HL formed on the one side in the lateral direction of the diaper 1. Then, the leg opening 1HL on the one side is arranged at the joint of the right leg of the wearer, that is to say at the same position as the one leg in the worn state. Thereafter, the operator putting on the diaper on pulls the other-side end portion of the front waist portion 30 toward laterally the other side by his/her hand, and then holds it in that state. The operator then pulls the fastening member 40 (the other-side end portion of the back waist portion 20) toward laterally the other side by his/her other hand, and wraps it around to the front side of the front waist portion 30. Then, the operator fastens the fastening portion 41 to the target region 34 of the front waist portion 30. Thus, the leg opening on the other side is formed, putting the diaper 1 on the wearer.

With this putting-on method, the fastening portion 41 is fastened to the target region 34 while the one of the wearer's legs has been inserted through the leg opening 1HL on the one side, thus making it possible to form the leg opening 1HL on the other side and the waist opening 1HB at the same time. This makes it possible to put on the diaper 1 easily even if the wearer (a newborn infant or an infant) is wriggling their legs.

In such an operation for putting on the diaper 1, the absorbent main body 10 joined to the front waist portion 30 shifts in the lateral direction according to laterally stretching of the front waist portion 30 when the front waist portion 30 (the back waist portion 20) is being pulled toward the lateral other side. For this reason, in the diaper 1, in the state where one of wearer's legs has been inserted into the leg opening 1HL on the lateral one side, the lateral center line RC of the back waist portion 20 is located on the other side relative to the lateral center line AC of the absorbent main body 10.

When one of wearer's legs has been inserted into the leg opening 1HL, the center line AC of the absorbent main body 10 is located on the one side (the right leg side) of the center line BC that indicates the center of the body of the wearer. In this state, the fastening member 40 is pulled to the other side, and the fastening portion 41 is fastened to the front waist portion 30, forming the diaper 1 into a shape when it is worn. Then, the back waist portion 20 stretches in the lateral direction, and the absorbent main body 10 moves to the other side (the left leg side). Accordingly, the center line AC and the center line BC approach each other, and the absorbent main body 10 can be brought near the center of the body of the wearer.

### Arrangement of Elastic Region

In the foregoing operation for putting on the diaper 1, if the position of the absorbent main body 10 is not able to be precisely adjusted to the center of the wearer's body, there is a possibility that the absorbent body 11 does not fit the wearer's body, causing leakage of excrement and/or giving discomfort to the wearer. In the present embodiment, adjusting the arrangement of the elastic regions X and Y of the diaper 1 makes it possible to move precisely the absorbent main body 10 to the center of the wearer's body when putting on the diaper 1. The arrangement of the elastic regions X and Y of the diaper 1 will be described below in detail.

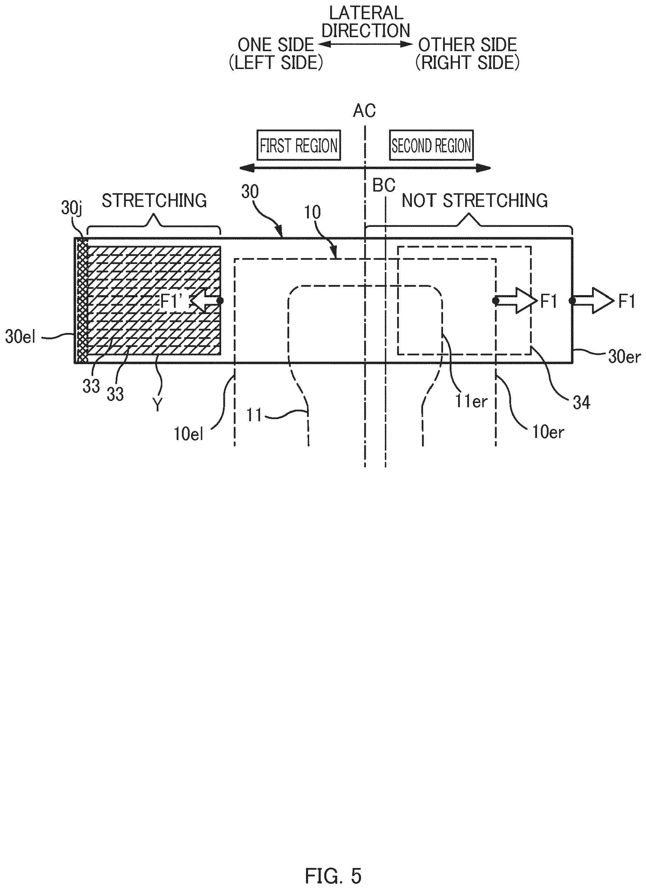

First, the side of the front waist portion 30 will be described below. FIG. 5 is a diagram illustrating a force exerted on the waist portion 30 when the diaper 1 is put on. FIG. 5 shows a state where the front waist portion 30 is pulled toward the lateral other side when the diaper 1 is put on. A region of the front waist portion 30 on the one side relative to the lateral center of the absorbent main body 10 (the center line AC) is defined as a first region. Similarly, a region of the front waist portion 30 on the other side relative to the lateral center of the absorbent main body 10 (the center line AC) is defined as a second region.

In the first region of the front waist portion 30, the elastic region Y is formed of a plurality of the elastic strings 33 being capable of stretching/contracting in the lateral direction. As described in FIG. 3, in the present embodiment, the elastic region Y is provided in a region from the lateral other-side end (inward end) of the joining region 30j and to the left end 10el of the absorbent main body 10. In the second region of the front waist portion 30, no elastic region is formed. That is, the entirety of the second region is a non-elastic (not stretching/contracting) region.

When putting on the diaper 1, while the laterally one-side end 30el of the front waist portion 30 being fixed to the wearer's body, the laterally other-side end 30er of the front waist portion 30 is pulled to the other side. Since the second region does not stretch/contract, force F1 for pulling the end 30er to the other side is transmitted, almost without changing, to the absorbent main body 10 through the second region (non-elastic region) of the front waist portion 30. Consequently, the other-side end 10er of the absorbent main body 10 is pulled at the force F1 to the other side in the lateral direction. This moves the absorbent main body 10 toward the lateral other side, and the center line AC of the absorbent main body 10 moves closer to the center line BC of the wearer's body.

According to pulling the end 30el to the other side, the elastic region Y of the first region is stretched in the lateral direction, producing a force F1' for contracting the first region in the lateral direction. With the contraction force, the one-side end 10el of the absorbent main body 10 is pulled to the other side in the lateral direction. That is, when the diaper 1 is put on, a force for pulling to both lateral sides is exerted on the absorbent main body 10. Accordingly, the absorbent main body 10 enlarges in the lateral direction, making it difficult to produce creases or the like on the absorbent body 11. This makes it easier to fit the absorbent body 11 to the wearer's body, making it easier to suppress troubles such as leakage of excrement. Even with a diaper 1 which is asymmetric on the left and right, the diaper 1 is easily balanced on the left and right sides when being put on, giving the good fit to the wearer.

As mentioned above, pulling the end 30er of the front waist portion 30 alone sufficiently makes it possible to easily align the absorbent main body 10 with the center of the wearer's body. In the pulling, adjusting the force F1 for pulling the end 30er can make slight change in the amount of movement of the absorbent main body 10. This makes it easier to improve the fit of the absorbent main body 10 when the diaper 1 is put on. And, the lateral other-side end portion of the front waist portion 30 including the end 30er is a non-elastic region. This makes it easier to hold and pull the non-elastic region, improving ease of putting on. Consequently, the operation for putting on the diaper 1 becomes comfortable.

As mentioned above, in the present embodiment, since no elastic region is provided in the second region between the other-side end 10er of the absorbent main body 10 and the other-side end 30er of the front waist portion 30, this makes it possible to move precisely the absorbent main body 10 in the lateral direction. However, the entirety of the second region is not necessary to be non-elastic. It is sufficient that no elastic region is provided in at least a region located on the other side relative to the other-side end 10er of the absorbent main body 10. This is because a region that does not stretch/contract can transmit the force F1 for pulling the front waist portion 30 directly to the absorbent main body 10, moving the absorbent main body 10.

Note that, for adjusting the position of the absorbent body 11 relative to the wearer's body, the second region may have no elastic region on the other side relative to the other-side end 11er of the absorbent body 11. Similarly, in the first region, the elastic region Y may be provided in a region on the one side relative to the one-side end 11el of the absorbent body 11. Even with such a configuration, it is possible to enlarge the absorbent body 11 and to precisely adjust the position of the absorbent body 11.

In the present embodiment, the target region 34 is provided in the second region of the front waist portion 30, and the entirety of the second region is non-elastic region and is difficult to stretch/contract. Accordingly, the target region 34 is likely to maintain its plain shape, and is less likely to crease. Further, the fastening projections of the fastening portion 41 are easy to be fastened to the surface of the target region 34, and after fastening, these components are difficult to be pulled apart. This makes it difficult for the diaper 1 to shift even when the wearer (e.g., an infant) moves his/her body, making it easier to maintain the good fit.

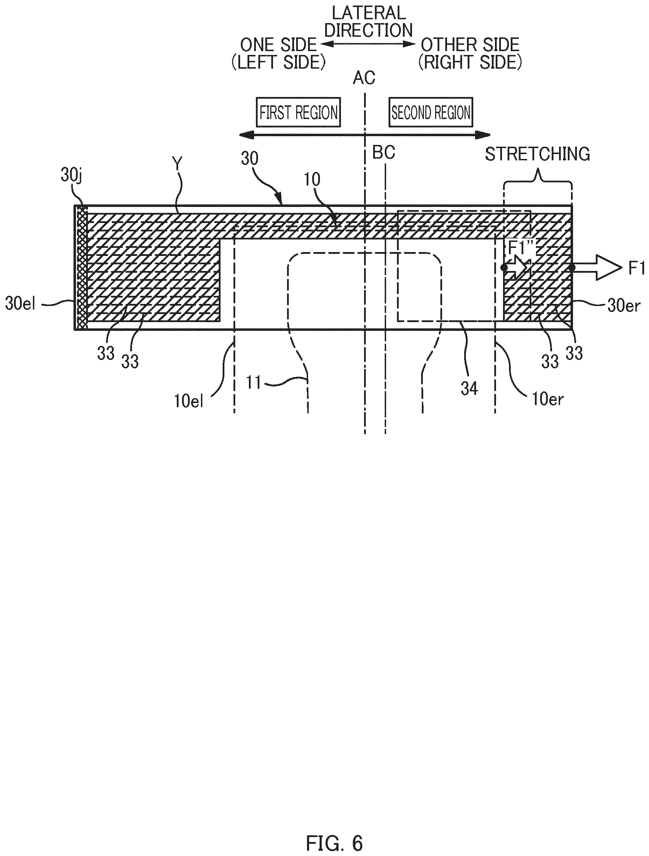

As a modified example, a case where non-elastic region is not provided in an appropriate part of the front waist portion 30 will be described below. FIG. 6 is a diagram illustrating a force exerted on the waist portion 30 when the diaper according to the modified example is put on. In the modified example, the upper part of the front waist portion 30 has an elastic region throughout a region from laterally one-side end 30el to the other-side end 30er, and does not have a non-elastic region. When putting on such a diaper, the front waist portion 30 is pulled to the lateral direction one side, and thereby a region of the second region between the other-side end 10er of the absorbent main body 10 and the other-side end 30er of the front waist portion 30 stretches/contracts. Accordingly, the force F1 for pulling the front waist portion 30 in the lateral direction is used for stretching the front waist portion 30, and the force F1" for pulling the other-side end 10er of the absorbent main body 10 is weaker than the force F1 (F1 > F1"). That is, the force F1 for pulling the front waist portion 30 in the lateral direction is not transmitted sufficiently to the absorbent main body 10, and the center line AC of the absorbent main body 10 becomes less likely to move to the center line BC of the wearer's body, making it difficult to realize the good fit. Accordingly, as in the present embodiment, it is preferable that the first region includes an elastic region in its predetermined region, and the second region does not include an elastic region in its predetermined region.

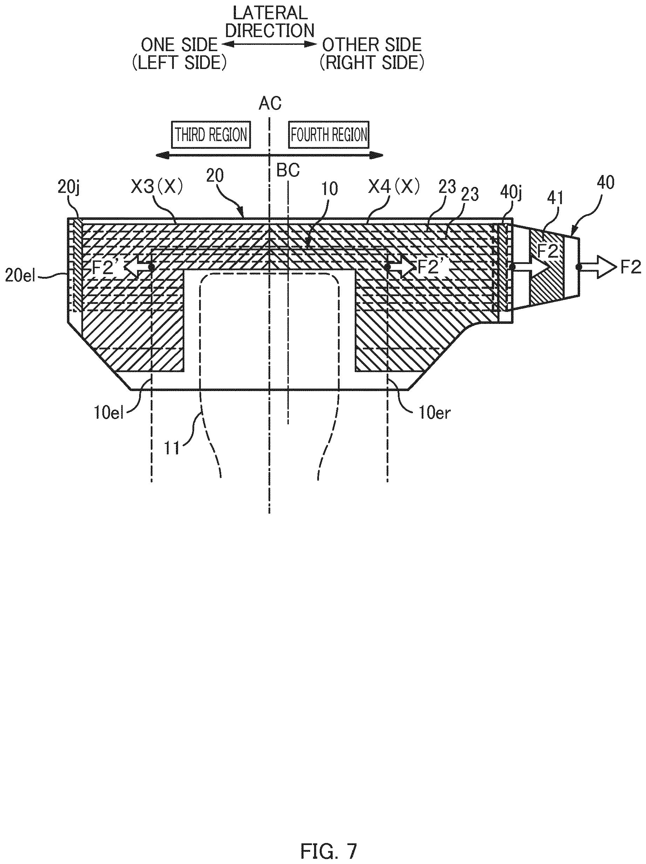

Next, the side of the back waist portion 20 will be described below. FIG. 7 is a diagram illustrating a force exerted on the back waist portion 20 when the diaper 1 is put on. FIG. 6 shows a state where the back waist portion 20 is pulled toward the lateral other side when the diaper 1 is put on. A region of the back waist portion 20 on the one side relative to the lateral center of the absorbent main body 10 (the center line AC) is defined as a third region. Similarly, a region of the back waist portion 20 on the other side relative to the lateral center of the absorbent main body 10 (the center line AC) is defined as a fourth region.

In the third region of the back waist portion 20, the elastic region X3 is formed of a plurality of the elastic strings 23 being capable of stretching/contracting in the lateral direction. In FIG. 7, the elastic region X3 extends from the lateral other-side end (inward end) of the joining region 20j to the center line AC. Similarly, in the fourth region, the elastic region X4 is formed of a plurality of elastic strings 23 being capable of stretching/contracting in the lateral direction. In FIG. 7, the elastic region X4 extends from the center line AC to the lateral one-side end (inward end) of the fixing region 40j. In the elastic region X3 and the elastic region X4, the elastic region X is formed throughout the entire lateral region of the back waist portion 20 (see FIG. 3).

When the diaper 1 is put on, while the laterally one-side end 20el of the back waist portion 20 being fixed to the wearer's body, the fastening member 40 located on the lateral other side of the back waist portion 20 is pulled to the other side. By force F2 for pulling the fastening member 40, the elastic regions X3 and X4 are stretched in the lateral direction, producing stretching force on the third region and the fourth region. Consequently, laterally outward force F2' is exerted on the lateral ends of the absorbent main body 10. The absorbent body 11 enlarges in the lateral direction, and stretchability is given to the waist opening 1HB, making it easier for the absorbent body 11 to fit to the wearer's body.

For enlarging the absorbent main body 10, it is sufficient that, similar to the description of the front waist portion 30, the elastic region X3 is provided in at least the third region of the back waist portion 20 from the center line AC to the lateral one side. However, the back waist portion 20 is a portion wrapping a wide area of the wearer's waist on the side of the wearer's buttocks when the diaper 1 is put on. Accordingly, in order to make it difficult for positional shift of the diaper 1 to occur, it is necessary that the waist opening 1HB has sufficient stretchability. For this purpose, in the diaper 1, the elastic region X4 is provided in the fourth region from the center line AC to the lateral other side so that stretchability is exerted on a wide range of the back waist portion 20. In the present embodiment, pulling the second region (non-elastic region) of the front waist portion 30 can adjust the lateral position of the absorbent main body 10. Accordingly, even though the fourth region (the elastic region X4) of the back waist portion 40 has stretchability, it is possible to align the position of the absorbent main body 10 with the center of the wearer's body.

In the diaper 1, the elastic region X3 and the elastic region X4 are continuous to each other. That is, the lateral stretching force of the elastic region X3 and the lateral stretching force of the elastic region X4 work together, ensuring stretchability throughout the entirety of the back waist portion 20. This makes it easier for the stretching forces to be exerted evenly. This makes it difficult to cause local variation of the stretching proportion and distortion which will cause when the elastic regions X3 and X4 stretch. And, this suppresses local tightness around the wearer's back waist when the diaper 1 is put on. Also, the back waist portion 20 becomes likely to fit on wearer's back, improving the fit of the diaper 1 when it is put on.

Further, in the diaper 1, the elastic region X3 and the elastic region Y are substantially continuous to each other. That is, the stretching force of the elastic region X3 of the back waist portion 20 and the stretching force of the elastic region Y of the front waist portion 20 work together, ensuring stretchability throughout the entire circumference of the waist opening 1HB of the diaper 1. This makes it easier for the stretching forces to be exerted evenly. This makes it difficult to cause local variation of the stretching proportion and distortion which will cause the elastic regions X3 and X4 and the elastic region Y stretch. And, this suppresses local tightness around the wearer's waist when the diaper 1 is put on, further improving the fit.

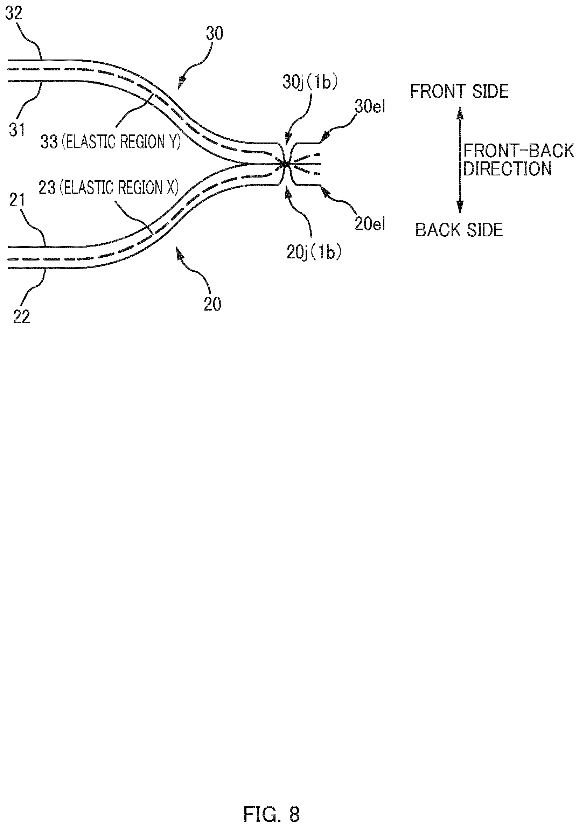

Note that a state where the elastic region X3 and the elastic region Y are substantially continuous to each other means that a range of the elastic region X3 in which stretching force is exhibited is continuous to a range of the elastic region Y in which stretching force is exhibited. FIG. 8 is a schematic sectional view illustrating continuity between the elastic region X3 and the elastic region Y. In the region of the lateral one-side end portion of the diaper 1, FIG. 8 shows the vicinity of the joining regions 30j and 40j viewed from above. In FIG. 8, the front waist portion 30 and the back waist portion 20 are joined in the first joining portion 1b (30j, 40j). Here, the elastic strings 33 provided in the elastic region Y is not necessarily configured to be as a single unit with the elastic strings 23 provided in the elastic region X3. But, since the elastic strings 33 and 22 are pressed together by the first joining portion 1b, stretching force of the elastic region X3 and stretching force of the elastic region Y work together. This makes it possible to exert stretching force on the entirety of the wearer's waist.

No elastic region is provided in the fastening member 40 (the fastening portion 41) joined to the other-side end portion of the back waist portion 20. That is, a lateral region on the other side relative to the elastic region X4 of the fourth region is a non-elastic region. The fastening member 40 is a part to be held for pulling the back waist portion 20 to the lateral other side when the diaper 1 is put on. Accordingly, if the part does not stretch/contract, it is easy to handle it. In addition, since the fastening member 40 does not stretch/contract, a force for pulling the fastening member 40 in the lateral direction is easy to be directly transmitted to the elastic region X4, making it possible to facilitate an operation of putting on the diaper. Further, since the fastening member 40 does not stretch/contract, this makes it easier for the fastening portion 41 provided in the fastening member 40 to maintain its planer shape. Accordingly, it is possible to securely fasten the fastening portion 41 to the target region 34.

In the diaper 1, there is a part in which the leg-gather elastic member 16 overlaps the fourth region of the back waist portion 20 (see FIG. 2). That is, the range on which the stretchability of the leg-gather elastic member 16 works overlaps in the longitudinal direction at least a part of the fourth region. With such a configuration, in the operation for putting on the diaper 1, when the back waist portion 20 is pulled to the lateral other side, the leg-gather elastic member 16 is pulled together with the back waist portion 20, pulling up the leg gather LG longitudinally upward and laterally to the other side. On the side of the wearer's buttocks (the fourth region), this makes the leg opening 1HL easier to fit the wearer's buttocks. As mentioned above, since the absorbent main body 10 (the center line AC) moves to the other side in the lateral direction when the diaper 1 is put on, the leg gather LG is more likely to crease due to being pressed by the absorbent main body 10. But, by stretching the leg gather LG due to the foregoing pulling up, creases produced on the leg gather LG are stretched, making it possible to improve the fit to the wearer's buttocks.

In the diaper 1, the elastic strings 23b provided in the lower part of the fourth region are arranged so as to intersect the leg-gather elastic member 16, and therefore the lateral stretching force of the elastic string 23b is also exerted on the leg-gather elastic member 16. This further increases pulling up of the leg gather LG.

In the diaper 1, the longitudinal length H20 of the back waist portion 20 is larger than the longitudinal length H30 of the front waist portion 30. Accordingly, when the diaper 1 is put on, the area of a part of the wearer's body covered with the back waist portion 20 is larger than the area of a part of the wearer's body covered with the front waist portion 30. That is, it is possible to cover reliably a region of the wearer's buttocks having a large area. Due to elastic force of the elastic region X through the lateral entirety of the back waist portion 20, the back waist portion 20 having a large area is stretched as a whole. This makes it difficult for the back waist portion 20 to crease even though the back waist portion 20 has a large area, making it possible to give the good fit to the wearer.

Note that in the above embodiment, the comparison of the lengths H20, H30, and the like shown in FIG. 2 is made with stretching the back waist portion 20, and the front waist portion 30 (and the absorbent main body 10). This "stretching" state is a state where the absorbent main body 10 is stretched in the longitudinal direction without creases, and where the back waist portion 20 and the front waist portion 30 are stretched in the lateral direction without creases. More specifically, it is a state as follow: the absorbent main body 10 is stretched in the longitudinal direction such that the longitudinal dimension thereof reaches a length equal or close to the longitudinal dimension of the top face sheet 12; the back waist portion 20 is stretched in the lateral direction such that the lateral dimension thereof is a length equal or close to the lateral dimension of the skin-side member 21 and the lateral dimension of the non-skin-side member 22; and the front waist portion 30 is stretched in the lateral direction such that the lateral dimension thereof is a length equal or close to the lateral dimension of the skin-side member 31 and the lateral dimension of the non-skin-side member 32.

### Other Embodiments

Although an embodiment of the present invention has been described above, the above embodiment is for facilitating the understanding of the present invention, and is not to be construed as limiting the present invention. The present invention can be modified, improved, etc. without departing from the gist of the present invention, and equivalents of the present invention are also encompassed within the present invention. For example, the invention can be altered as described below.

Although the above embodiment illustrates the so-called three piece type of disposable diaper 1 as an example of the absorbent article, there is no limitation whatsoever to this. For example, the absorbent article may be a two piece type of disposable diaper including: a first component is an exterior sheet including a back waist portion and a front waist portion that are connected via a crotch portion as a single unit; and a second component is an absorbent main body that is fixed to the skin-side surface of the exterior sheet.

The above embodiment describes an example in which the target region 34 has loops, the fastening portion 41 has hooks, and the fastening portion 41 is fastened to the target region 34 by the loops becoming caught on the hooks. However, the configurations of the target region 34 and the fastening portion 41 are not limited to the above example. For example, at least one of the target region 34 and the fastening portion 41 may be provided with adhesiveness on its surface, and they may be fastened by adhering this adhesive member to the surface of the other member.

The above embodiment describes the state where the fastening member 40 projects in a lateral direction from the back waist portion 20 when the diaper is put on. However, the fastening member 40 may be folded when the disposable diaper 1 is manufactured, or the fastening member 40 may be provisionally connected to the front waist portion 30 by perforations.

In the above embodiment, although the elastic strings 23 and 33 are used as the elastic members, there is no limitation to this. It is possible to use a nonwoven fabric that has stretchability, for example.

In the above embodiment, although the elastic strings 23 and 33 are not provided in the overlapping regions of the back waist portion 20 and the front waist portion 30 in which they overlap with the absorbent body 11, there is no limitation to this. The elastic strings 23 and 33 may be provided in the regions overlapping with the absorbent body 11. By not providing the elastic strings 23 and 33 in the regions overlapping with the absorbent body 11, it is possible to reduce the risk of the absorbent body 11 deformed due to stretching and contracting of the elastic strings 23 and 33. However, by providing the elastic strings 23 and 33 in the regions overlapping with the absorbent body 11, it is possible to improve the fit of the absorbent main body 10 through stretching force.

### [Reference Signs List]

1 diaper (absorbent article),
1b first joining portion, 2b second joining portion,
1HB waist opening, 1HL leg opening,
10 absorbent main body (crotch portion),
10a one end portion, 10b other end portion, 10er end, 10el end,
11 absorbent body, 11er end, 12 top face sheet, 13 back-face sheet,
16 leg-gather elastic member,
20 back waist portion, 20a upper end, 20b lower end, 20bs straight portion,
20bl inclined portion (one-side inclined portion), 20br inclined portion (other-side inclined portion),
20er end, 20el end, 20j joining region,
21 skin-side member, 22 non-skin-side member, 23 elastic string (elastic member),
30 front waist portion, 30a upper end, 30b lower end,
30er end, 30el end, 30j joining region,
31 skin-side member, 32 non-skin-side member, 33 elastic string (elastic member),
34 target region,
40 fastening member, 40j fixing region,
41 fastening portion, H41 length (longitudinal direction), W41 width (lateral direction),
X elastic region, Xe1 one-side end, Xe2 other-side end,
X3 elastic region, X4 elastic region, Y elastic region

## Claims

1. An absorbent article (1) having a longitudinal direction, a lateral direction intersecting the longitudinal direction, and a front-back direction intersecting the longitudinal direction and the lateral direction,
the absorbent article comprising:
a front waist portion (30) extending along the lateral direction;
a back waist portion (20) extending along the lateral direction; and
an absorbent main body (10) including an absorbent body (11) that absorbs liquid, and being provided between the front waist portion (30) and the back waist portion (20),
a one-side end portion of the back waist portion (20) on a one side in the lateral direction being joined by a first joining portion (1b) to a one-side end portion of the front waist portion (30) on the one side in the lateral direction,
the back waist portion (20) including a fastening portion (41) on another side in the lateral direction,
the fastening portion (41) being fastened to the front waist portion (30) when putting on the absorbent article,
the front waist portion (30) having a first region located on the one side relative to a lateral center (AC) of the absorbent main body (10),
the front waist portion having a second region located on the other side relative to the lateral center (AC) of the absorbent main body,
an elastic region (Y) stretching/contracting in the lateral direction,
the elastic region (Y) being provided in at least a part of the first region, **characterized in that**
the elastic region is not provided in a region of the second region, thereby forming a non-elastic region which is provided in at least a region located on the other side relative to an end (10er) of the absorbent main body on the other side.

2. An absorbent article according to claim 1, wherein
the back waist portion (20) has a third region located on the one side relative to the lateral center (AC) of the absorbent main body, and
the elastic region (X) is provided in at least a part of the third region.

3. An absorbent article according to claim 2, wherein
the back waist portion (20) has a fourth region located on the other side relative to the lateral center (AC) of the absorbent main body, and
the elastic region is provided in at least a part of the fourth region.

4. An absorbent article according to claim 3, wherein
the elastic region (X4) is not provided in a region located on the other side relative to the elastic region that is provided in the fourth region.

5. An absorbent article according to claim 3 or 4, wherein
the elastic region (X3) provided in the third region and the elastic region (X4) provided in the fourth region are continuous in the lateral direction to each other.

6. An absorbent article according to any one of claims 2 to 5, wherein
the elastic region (Y) provided in the first region and the elastic region (X3) provided in the third region are substantially continuous in the lateral direction to each other.

7. An absorbent article according to any one of claims 1 to 6, wherein
the elastic region (Y) is not provided at all in any region of the second region.

8. An absorbent article according to claim 7, wherein
on a non-skin side of the front waist portion (30), a target region (34) is provided in a predetermined region of the second region, and
the target region is a region to which the fastening portion (41) is fastened.

9. An absorbent article according to any one of claims 1 to 8, wherein
a leg-gather elastic member (16) is provided on each of lateral end portions of a crotch portion,
the leg-gather elastic member (16) being capable of stretching/contracting in the longitudinal direction, and
a range on which an elastic force of the leg-gather elastic member is exerted overlaps in the longitudinal direction at least a partial region of the back waist portion (20) on the other side relative to the lateral center of the absorbent main body (10).

10. An absorbent article according to any one of claims 1 to 9, wherein
a longitudinal length (H20) of the back waist portion (20) is larger than a longitudinal length (H30) of the front waist portion (30).

## Patentansprüche

1. Absorbierender Artikel (1), der Folgendes aufweist: eine Längsrichtung, eine laterale Richtung, die die Längsrichtung kreuzt, und eine Vorderseite-Hinterseite-Richtung, die die Längsrichtung und die laterale Richtung kreuzt,
wobei der absorbierende Artikel Folgendes umfasst:
ein vorderes Taillenteil (30), das sich entlang der lateralen Richtung erstreckt;
ein hinteres Taillenteil (20), das sich entlang der lateralen Richtung erstreckt; und
einen absorbierenden Hauptkörper (10), der einen Saugkörper (11) einschließt, der Flüssigkeit absorbiert, und zwischen dem vorderen Taillenteil (30) und dem hinteren Taillenteil (20) bereitgestellt ist,
wobei ein einseitiges Endteil des hinteren Taillenteils (20) auf einer einen Seite in der lateralen Richtung durch ein erstes Verbindungsteil (1b) mit einem einseitigen Endteil des vorderen Taillenteils (30) auf der einen Seite in der lateralen Richtung verbunden ist,
das hintere Taillenteil (20) ein Befestigungsteil (41) auf einer anderen Seite in der lateralen Richtung einschließt,
wobei das Befestigungsteil (41) an dem vorderen Taillenteil (30) befestigt wird, wenn der absorbierende Artikel angelegt wird,
wobei das vordere Taillenteil (30) einen ersten Bereich aufweist, der sich auf der einen Seite, in Bezug auf eine laterale Mitte (AC) des absorbierenden Hauptkörpers (10), befindet,
das vordere Taillenteil einen zweiten Bereich aufweist, der sich auf der anderen Seite, in Bezug auf die laterale Mitte (AC) des absorbierenden Hauptkörpers, befindet,
ein elastischer Bereich (Y) sich in der lateralen Richtung dehnt/zusammenzieht,
der elastische Bereich (Y) in mindestens einem Teil des ersten Bereichs bereitgestellt ist, **dadurch gekennzeichnet, dass**
der elastische Bereich nicht in einem Bereich des zweiten Bereichs bereitgestellt ist, wodurch ein nichtelastischer Bereich ausgebildet wird, der in mindestens einem Bereich, der sich auf der anderen Seite, in Bezug auf ein Ende (10er) des absorbierenden Hauptkörpers, befindet, auf der anderen Seite bereitgestellt ist.

2. Absorbierender Artikel nach Anspruch 1, wobei
das hintere Taillenteil (20) einen dritten Bereich aufweist, der sich auf der einen Seite, in Bezug auf die laterale Mitte (AC) des absorbierenden Hauptkörpers, befindet, und
der elastische Bereich (X) in mindestens einem Teil des dritten Bereichs bereitgestellt ist.

3. Absorbierender Artikel nach Anspruch 2, wobei
das hintere Taillenteil (20) einen vierten Bereich aufweist, der sich auf der anderen Seite, in Bezug auf die laterale Mitte (AC) des absorbierenden Hauptkörpers, befindet, und
der elastische Bereich in mindestens einem Teil des vierten Bereichs bereitgestellt ist.

4. Absorbierender Artikel nach Anspruch 3, wobei
der elastische Bereich (X4) nicht in einem Bereich bereitgestellt ist, der sich auf der anderen Seite, in Bezug auf den elastischen Bereich, befindet, der in dem vierten Bereich bereitgestellt ist.

5. Absorbierender Artikel nach Anspruch 3 oder 4, wobei
der elastische Bereich (X3), der in dem dritten Bereich bereitgestellt ist, und der elastische Bereich (X4), der in dem vierten Bereich bereitgestellt ist, in der lateralen Richtung zueinander kontinuierlich verlaufen.

6. Absorbierender Artikel nach einem der Ansprüche 2 bis 5, wobei
der elastische Bereich (Y), der in dem ersten Bereich bereitgestellt ist, und der elastische Bereich (X3), der in dem dritten Bereich bereitgestellt ist, in der lateralen Richtung im Wesentlichen zueinander kontinuierlich verlaufen.

7. Absorbierender Artikel nach einem der Ansprüche 1 bis 6, wobei
der elastische Bereich (Y) in keinem Bereich des zweiten Bereichs bereitgestellt ist.

8. Absorbierender Artikel nach Anspruch 7, wobei
auf einer Nicht-Hautseite des vorderen Taillenbereichs (30) ein Zielbereich (34) in einem vorgegebenen Bereich des zweiten Bereichs bereitgestellt ist und
der Zielbereich ein Bereich ist, an dem das Befestigungsteil (41) befestigt wird.

9. Absorbierender Artikel nach einem der Ansprüche 1 bis 8, wobei
ein elastisches Beinraffungselement (16) an jedem der lateralen Endteile eines Schrittteils bereitgestellt ist,
das elastische Beinraffungselement (16) sich in der Längsrichtung dehnen/zusammenziehen kann und
eine Weite, auf die eine elastische Kraft des elastischen Beinraffungselements ausgeübt wird, in der Längsrichtung mit mindestens einem Teilbereich des hinteren Taillenbereichs (20) auf der anderen Seite, in Bezug auf die laterale Mitte des absorbierenden Hauptkörpers (10), überlappt.

10. Absorbierender Artikel nach einem der Ansprüche 1 bis 9, wobei
eine Länge in Längsrichtung (H20) des hinteren Taillenteils (20) größer ist als eine Länge in Längsrichtung (H30) des vorderen Taillenteils (30).

## Revendications

1. Article absorbant (1) ayant une direction allant dans le sens longitudinal, une direction allant dans le sens latéral croisant la direction allant dans le sens longitudinal, et une direction allant dans le sens avant-arrière croisant la direction allant dans le sens longitudinal et la direction allant dans le sens latéral,
l'article absorbant comportant :
une partie avant au niveau de la taille (30) s'étendant le long de la direction allant dans le sens latéral ;
une partie arrière au niveau de la taille (20) s'étendant le long de la direction allant dans le sens latéral ; et
un corps principal absorbant (10) comprenant un corps absorbant (11) qui absorbe le liquide, et étant mis en œuvre entre la partie avant au niveau de la taille (30) et la partie arrière au niveau de la taille (20),
une partie d'extrémité d'un côté de la partie arrière au niveau de la taille (20) sur un dit un côté dans la direction allant dans le sens latéral qui est assemblée par une première partie d'assemblage (1b) à une partie d'extrémité d'un côté de la partie avant au niveau de la taille (30) sur ledit un côté dans la direction allant dans le sens latéral,
la partie arrière au niveau de la taille (20) comprenant une partie d'attache (41) sur un autre côté dans la direction allant dans le sens latéral,
la partie d'attache (41) étant attachée à la partie avant au niveau de la taille (30) quand l'article absorbant est mis en place,
la partie avant au niveau de la taille (30) ayant une première région située sur ledit un côté par rapport à un centre latéral (AC) du corps principal absorbant (10),
la partie avant au niveau de la taille ayant une deuxième région située sur ledit autre côté par rapport au centre latéral (AC) du corps principal absorbant,
une région élastique (Y) s'étirant/se contractant dans la direction allant dans le sens latéral,
la région élastique (Y) étant mise en œuvre dans au moins une partie de la première région, **caractérisé en ce que**
la région élastique n'est pas mise en œuvre dans une région de la deuxième région, pour de ce fait former une région non élastique qui est mise en œuvre dans au moins une région située sur ledit autre côté par rapport à une extrémité (10er) du corps principal absorbant sur ledit autre côté.

2. Article absorbant selon la revendication 1, dans lequel
la partie arrière au niveau de la taille (20) a une troisième région située sur ledit un côté par rapport au centre latéral (AC) du corps principal absorbant, et
la région élastique (X) est mise en œuvre dans au moins une partie de la troisième région.

3. Article absorbant selon la revendication 2, dans lequel
la partie arrière au niveau de la taille (20) a une quatrième région située sur ledit autre côté par rapport au centre latéral (AC) du corps principal absorbant, et
la région élastique est mise en œuvre dans au moins une partie de la quatrième région.

4. Article absorbant selon la revendication 3, dans lequel
la région élastique (X4) n'est pas mise en œuvre dans une région située sur ledit autre côté par rapport à la région élastique qui est mise en œuvre dans la quatrième région.

5. Article absorbant selon la revendication 3 ou la revendication 4, dans lequel
la région élastique (X3) mise en œuvre dans la troisième région et la région élastique (X4) mise en œuvre dans la quatrième région sont continues dans la direction allant dans le sens latéral l'une par rapport à l'autre.

6. Article absorbant selon l'une quelconque des revendications 2 à 5, dans lequel
la région élastique (Y) mise en œuvre dans la première région et la région élastique (X3) mise en œuvre dans la troisième région sont sensiblement continues dans la direction allant dans le sens latéral l'une par rapport à l'autre.

7. Article absorbant selon l'une quelconque des revendications 1 à 6, dans lequel
la région élastique (Y) n'est pas du tout mise en œuvre dans quelque région que ce soit de la deuxième région.

8. Article absorbant selon la revendication 7, dans lequel
sur un côté non orienté vers la peau de la partie avant au niveau de la taille (30), une région cible (34) est mise en œuvre dans une région prédéterminée de la deuxième région, et
la région cible est une région au niveau de laquelle la partie d'attache (41) est attachée.

9. Article absorbant selon l'une quelconque des revendications 1 à 8, dans lequel
un élément élastique formant des fronces au niveau de la jambe (16) est mis en œuvre sur chacune des parties d'extrémité latérale d'une partie au niveau de l'entrejambe,
l'élément élastique formant des fronces au niveau de la jambe (16) étant en mesure de s'étirer/se contracter dans la direction allant dans le sens longitudinal, et
une plage sur laquelle une force élastique de l'élément élastique formant des fronces au niveau de la jambe est exercée chevauche dans la direction allant dans le sens longitudinal au moins une région partielle de la partie arrière au niveau de la taille (20) sur ledit autre côté par rapport au centre latéral du corps principal absorbant (10).

10. Article absorbant selon l'une quelconque des revendications 1 à 9, dans lequel
une longueur longitudinale (H20) de la partie arrière au niveau de la taille (20) est plus large qu'une longueur longitudinale (H30) de la partie avant au niveau de la taille (30).
